Europäisches Patentamt

European Patent Office

Office européen des brevets

(19)

(11) Veröffentlichungsnummer: **0 245 767**
**A2**

(12) **EUROPÄISCHE PATENTANMELDUNG**

(21) Anmeldenummer: **87106575.1**

(22) Anmeldetag: **06.05.87**

(51) Int. Cl.⁴: **G01N 33/52**

(30) Priorität: **13.05.86 DE 3616105**

(43) Veröffentlichungstag der Anmeldung:
**19.11.87 Patentblatt 87/47**

(84) Benannte Vertragsstaaten:
**BE CH DE FR GB IT LI NL SE**

(71) Anmelder: **Merck Patent Gesellschaft mit
beschränkter Haftung
Frankfurter Strasse 250
D-6100 Darmstadt(DE)**

(72) Erfinder: **Gunkel, Werner
Wingertsweg 33
D-6101 Rossdorf(DE)**
Erfinder: **Krauss, Helmuth, Dr.
Paul-Siebenweg 4
D-6140 Bensheim 3(DE)**

(54) **Teststreifen.**

(57) Die Erfindung betrifft Teststreifen zum Nachweis
von Inhaltsstoffen in Flüssigkeiten, die im wesentlichen aus einem festen Träger und mindestens einer
mit Reagenzien imprägnierten saugfähigen Matrix
bestehen. Die Matrizes sind durch Klebstoffstreifen
voneinander getrennt auf dem Träger fixiert und die
Klebstoffstreifen sind mit Folienstreifen abgedeck.

FIG.1

EP 0 245 767 A2

## Teststreifen

Die Erfindung betrifft Teststreifen zum Nachweis von Inhaltsstoffen in Flüssigkeiten, die im wesentlichen aus einem festen Träger und mindestens einer mit Reagenzien imprägnierten saugfähigen Matrix bestehen, wobei die Befestigung der Matrizes auf dem Träger außerhalb des zur Auswertung verwendeten Bereichs liegt.

Teststreifen zur qualitativen und in zunehmendem Maße in Verbindung mit Auswertegeräten auch zur quantitativen Bestimmung verschiedener Substanzen in Lösung sind seit langem bekannt. Solche Teststreifen bestehen in der Regel aus einem Kunststoffstäbchen, in dessen unterem Bereich die Matrizes oder Reaktionszonen in Form von mit Reagenzien getränkten Papieren, mit Reagenzien beschichteten Folien oder speziellen, das Reagenz beinhaltende Schichten befestigt sind. Zur Befestigung der Reaktionszonen auf dem Träger werden verschiedene Methoden angewandt. Sehr verbreitet ist z.B. die Befestigung der Reaktionszonen mit Hilfe von unter der Zone aufgetragenen Klebstoffen. Probleme bereitet hierbei - insbesondere bei den oft sehr empfindlichen Reagenzien und Enzymen, wie sie in der klinischen Chemie verwendet werden - die Auswahl von ausreichend inerten Klebstoffen. Eine weitere gebräuchliche Methode ist das mechanische Einklemmen der Reagenzzonen mit Hilfe von über die Zone gespannten Kunststoffnetzen (DE-PS 21 18 455). Nachteilig ist hierbei die visuelle Beeinträchtigung der Reaktionszonenoberfläche durch das Netz. Insbesondere bei der Geräteauswertung hat das Netz einen die Auswertung störenden Einfluß. Außerdem ist das seitliche Herausfallen der nur festgeklemmten Reaktionszonen bei der Anwendung nicht sicher zu verhindern.

Der vorliegenden Erfindung liegt die Aufgabe zugrunde, Teststreifen zur Verfügung zu stellen, deren Auswertezonen frei von Einflüssen der Befestigungselemente, wie Klebstoffe oder über die Zonen gespannte Netze, sind.

Gegenstand der Erfindung sind demnach Teststreifen zum Nachweis von Inhaltsstoffen in Flüssigkeiten, die im wesentlichen aus einem festen Träger (1) und mindestens einer mit Reagenzien imprägnierten saugfähigen Matrix (2) bestehen, die dadurch gekennzeichnet sind, daß die Matrizes durch Klebstoffstreifen (3) voneinander getrennt auf den Träger (1) fixiert und die Klebstoffstreifen (3) mit Folienstreifen (4) abgedeckt sind.

In einer weiteren Ausführungsform ist die dem Träger abgewandte Oberfläche der Matrizes mit einer im Bereich der Auswertezone gelochten Folie (5) abgedeckt. Die Folien (4, 5) können sowohl transparent als auch nicht transparent sein, vorzugsweise sind sie nicht transparent. Die Folien sollen mindestens 50 % der Auswertezone der Matrizes freilassen.

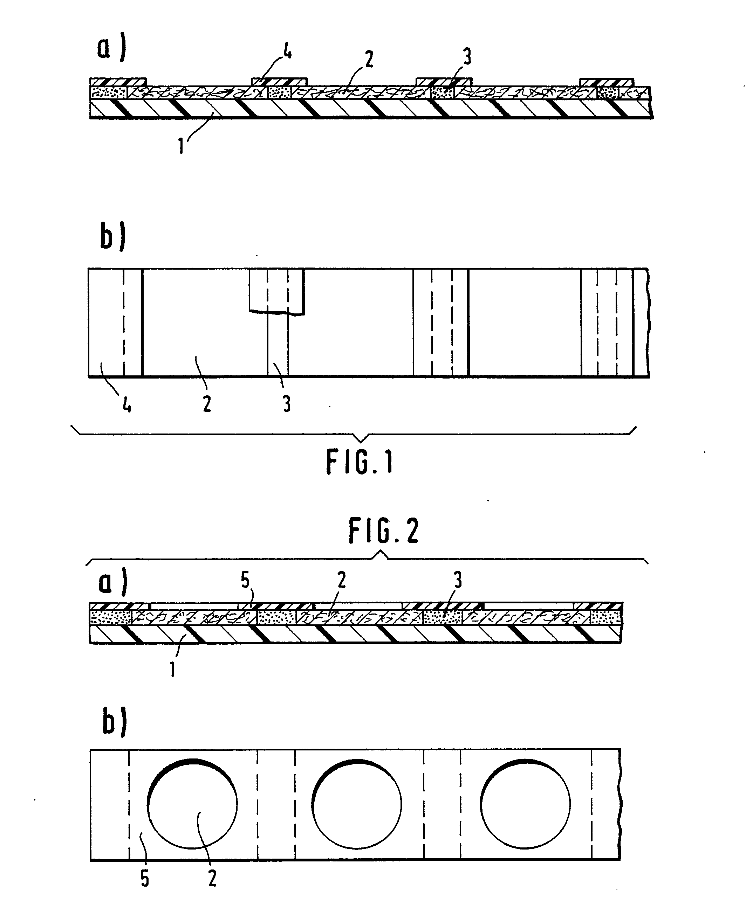

Die Abbildungen 1 und 2 zeigen den Aufbau der erfindungsgemäßen Teststreifen. Die jeweils mit a) bezeichneten Abbildungen 1 und 2 zeigen einen vergrößerten Querschnitt des unteren Teils eines erfindungsgemäßen Teststreifens, die mit b) bezeichneten Abbildungen eine entsprechende Aufsicht in vergrößerter Darstellung. Mit (1) ist der feste Träger, mit (2) die Matrizes bzw. Reaktionszonen, mit (3) der Klebstoffstreifen zwischen den Matrizes, mit (4) der Folienstreifen als Abdeckung über dem Klebstoffstreifen (3) und mit (5) die gelochte Folie bezeichnet.

Die Abbildung 1 zeigt einen Teststreifen, bei dem die Matrizes (2) mit Hilfe von dazwischen aufgetragenen Klebstoffstreifen (3) in ihrer Lage auf dem Träger (1) fixiert sind. Zur sicheren Befestigung auf dem Träger (1) und zur Abdeckung der vom Klebstoff beeinflußten Bereiche der Matrizes sind diese Bereiche mit Folienstreifen (4) versehen, die auch die Randbereiche der Matrizes noch überlappen.

Abbildung 2 zeigt eine Ausführungsform, bei der die gesamte Oberfläche mit einer jeweils im Bereich der Reaktionszonen gelochten Folie (5) überdeckt ist. Die Folie ist zwischen den Reaktionszonen mit dem Klebstoffstreifen (3) und im Griffbereich mit der Trägerfolie (1) verklebt. Die zur Auswertung verwendete Reagenzzone hat damit keinerlei Kontakt mit dem Klebstoff und ist an der Oberfläche völlig frei.

Als feste Träger (1) kommen die üblichen Folienstreifen aus Kunststoffen wie z.B. Polystyrol, Polyvinylchlorid, Polyester, Polyamide in Frage; jedoch sind auch Träger aus Metallfolien, z.B. aus Aluminium oder auch solche aus Glas gut geeignet.

Die mit Reagenzien imprägnierten saugfähigen Matrizes (2) bestehen z.B. aus Filterpapier, Vliesen, Gewebe aus Natur-oder Kunstfasern, porösen Gelen oder aus Verbunden dieser Materialien. Für spezielle Zwecke sind auch mit Reagenzien beschichtete Folien oder in Kunststoff inkorporierte Reagenzien gut geeignet.

Die Klebstoffstreifen (3) am oberen und unteren Ende der Matrix bzw. zwischen den einzelnen Matrizes bestehen aus den üblichen Schmelzklebern oder Kaltklebern. Zur einfachen und wirtschaftlichen Herstellung der Teststreifen nach der Erfindung werden vorzugsweise Schmelzkleber verwendet. Die Klebstoffstreifen sind etwa 0,5-3 mm, vorzugsweise 1-2 mm breit, und dienen vor allem dazu, die Matrizes in ihrer Lage auf dem festen Träger zu fixieren.

Sowohl die Klebstoffstreifen als auch die mit dem Klebstoff in Kontakt gekommenen oberen und unteren Randzonen der Matrizes werden erfindungsgemäß mit Folienstreifen (4) abgedeckt. Diese Folienstreifen bestehen vorzugsweise aus einem nicht transparenten, hydrophoben Material, wie Polyester oder Polystyrol oder auch aus hydrophobiertem Papier von 50-100 µm Dicke. Sie sollten die Randbereiche der Matrizes um etwa 0,5-1 mm überdecken und so mit dem festen Träger verbinden.

Die bei der Ausführungsform nach Abbildung 2 verwendete gelochte Folie (5) besteht aus dem gleichen Material wie die beschriebenen Folienstreifen. Die Form der Lochung ist nicht kritisch, sie kann rund, quadratisch, rechteckig oder vieleckig sein. Im Falle der runden Lochung sollte der Durchmesser des Loches etwa 1 mm kleiner sein als die Breite oder Länge der darunter liegenden Matrixfläche; in jedem Fall sollte die Folie mindestens 50 %, vorzugsweise bis zu 90 % der Matrixfläche, die sogenannte Auswertezone, freilassen.

Die Dimensionen der erfindungsgemäßen Teststreifen nach Abbildung 1 entsprechen denen handelsüblicher Teststreifen. Die Breite der Matrix beträg etwa 7,5 mm, der Abstand zwischen den einzelnen Matrizes, d.h. die Breite des Klebstoffstreifens, etwa 1,5 mm und der Folienstreifen etwa 3 mm. In diesem Falle sind z.B. 20 % einer quadratischen Matrix durch den Folienstreifen abgedeckt.

Die Herstellung der Teststreifen nach der Erfindung erfolgt auf einer üblichen Schmelzkleber-Siegelanlage. Auf die Trägerfolie (1) wird der verflüssigte Schmelzkleber (3) linienförmig aufgetragen. Zwischen die noch nicht ausgehärteten Klebstoffstreifen (3) werden die mit Reagenzien imprägnierten Matrixstreifen (2) eingelegt. Direkt dahinter werden die Folienstreifen (4) bzw. die gelochte Folie (5) zugeführt. Mit Hilfe einer Andruckwalze werden die einzelnen Teile auf die Trägerfolie gedrückt. Im Bereich des Klebstoffs entsteht auf diese Weise eine feste Verbindung zwischen Trägerfolie (1), Matrix (2) und Deckfolie (4) bzw. gelochter Folie (5). Das nach dieser Methode hergestellte Band wird anschließend wie üblich mit einem Querschneider in ca. 6-8 mm breite Streifen geschnitten.

## Ansprüche

1. Teststreifen zum Nachweis von Inhaltsstoffen in Flüssigkeiten, bestehend im wesentlichen aus einem festen Träger (1) und mindestens einer mit Reagenzien imprägnierten saugfähigen Matrix (2) dadurch gekennzeichnet, daß die Matrizes durch Klebstoffstreifen (3) voneinander getrennt auf dem Träger (1) fixiert und die Klebstoffstreifen (3) mit Folienstreifen (4) abgedeckt sind.

2. Teststreifen nach Anspruch 1, dadurch gekennzeichnet, daß die dem Träger abgewandte Oberfläche der Matrizes mit einer im Bereich der Auswertezone gelochten Folie (5) abgedeckt ist.

3. Teststreifen nach den Ansprüchen 1 und 2, dadurch gekennzeichnet, daß die Folien (4,5) mindestens 50 % der Auswertezone freilassen.

4. Teststreifen nach den Ansprüchen 1 bis 3, dadurch gekennzeichnet, daß die Folien (4, 5) nicht transparent sind.

a)

4   2   3

1

b)

4   2   3

FIG.1

FIG.2

a)

5   2   3

1

b)

5   2